# EUROPÄISCHE PATENTSCHRIFT

(11) **EP 2 589 348 B1**
(45) Veröffentlichungstag und Bekanntmachung des Hinweises auf die Patenterteilung: **06.12.2017**
(21) Anmeldenummer: 12189858.9
(22) Anmeldetag: 25.10.2012
(51) Int. Cl.: A61B 17/3205, A61B 17/50, A61B 17/34, A61B 17/064, A61B 17/068, A61N 1/05, A61B 18/00, A61B 17/29, A61B 17/30

(54) **Vorrichtung zur Explantation von Elektrodenleitungen**
Device for explanting electrode leads
Dispositif destiné à l'explantation de dérivations d'électrodes

(30) Priorität: 03.11.2011 DE 102011085683; 19.01.2012 US 201261588183 P
(43) Veröffentlichungstag der Anmeldung: 08.05.2013
(73) Patentinhaber: VascoMed GmbH, 79589 Binzen (DE)
(72) Erfinder: Geistert, Wolfgang, 79618 Rheinfelden (DE)
(74) Vertreter: Galander, Marcus

(56) Entgegenhaltungen:
- US-A- 5 697 936
- US-A- 5 779 715
- US-A- 6 033 402
- US-A1- 2005 192 591
- US-A1- 2006 116 689
- US-A1- 2008 255 597
- US-A1- 2010 198 229
- US-A1- 2011 106 099

## Beschreibung

Gegenstand der Patentanmeldung ist eine Vorrichtung zur Explantation von implantierten Elektrodenleitungen.

Der Einsatz von implantierbaren Elektrodenleitungen in der Medizin ist schon lange bekannt. Implantierbare Elektrodenleitungen werden insbesondere dazu eingesetzt, elektrische Implantate - in der Regel Herzschrittmacher, Defibrillatoren, Cardioverter und ähnliches - mit der elektromedizinisch zu behandelnden Stelle zu verbinden. Im seit langem bekannten Einsatzbereich ist diese Stelle das Herz, an oder in dem die Elektrodenleitungen befestigt werden. Durchgesetzt haben sich zwischenzeitlich in das Herzen führende Elektrodenleitungen, welche beispielsweise durch den rechten Vorhof in das Vorhofohr oder in die Spitze (Apex) der rechten Herzkammer geführt werden und dort entweder durch passive Verankerungen - so genannte "Tines" - oder mittels einer Einschraubung im Herzgewebe fixiert sind. Bei bestimmten Indikationen werden solche Elektrodenleitungen auch durch den Vorhof des Herzens in den Koronarsinus (ein Herzkranzgefäß, auch kurz "CS" genannt) geführt, um dort auf der linken Seite des Herzens eine Therapie durchzuführen.

Alle in dieser Patentanmeldung genannten Elektrodenleitungen umfassen einen lang gestreckten Elektrodenkörper, an dessen distalem Ende neben den soeben erwähnten beispielhaften Fixierungsmöglichkeiten auch mindestens ein Elektrodenpol vorhanden ist, welcher therapeutische Signale abgibt und/oder physiologische Signale abfühlt. Am dem distalen Ende entgegengesetzten proximalen Ende des Elektrodenkörpers befindet sich mindestens ein Stecksystem, mit dem die implantierte Elektrodenleitung an das elektrische Implantat angeschlossen ist. Im Folgenden bedeutet "distal" in Richtung des Explantationsortes weisend und "proximal" in Richtung des Operateurs weisend. Im Elektrodenkörper verlaufen in Längsrichtung elektrische Verbindungsleitungen, mit denen der mindestens eine Elektrodenpol am distalen Ende mit dem Stecksystem am proximalen Ende elektrisch verbunden ist. Des Weiteren kann die Elektrodenleitung ein in der Regel zentral verlaufendes durchgehendes Lumen aufweisen, welches am proximalen und gegebenenfalls am distalen Ende eine Verbindung mit der Umgebung aufweist. Dieses Lumen wird zur Führung der implantierbaren Elektrodenleitung entlang eines vorher gelegten Führungsdrahtes oder mit einem Mandrin genutzt. Der Elektrodenkörper kann auch noch andere Elemente umfassen, auf die hier jedoch nicht weiter eingegangen wird.

Unmittelbar nach der Implantation einer Elektrodenleitung beginnt ein natürlicher Prozess, der dafür sorgt, dass die Elektrodenleitung verwächst. Dabei wird sie in der Regel zumindest an der Elektrodenspitze von Gewebe umwachsen, was zu einer Fixierung führt. Jedoch ist es auch möglich, dass die Elektrodenleitung nicht nur an der Spitze, sondern undefiniert an ihrem Elektrodenkörper verwächst, beispielsweise an Stellen, an denen die Elektrodenleitung das umliegende Gefäß- oder Herzgewebe berührt. Dieser Verwachsungsprozess führt in der Regel dazu, dass die Elektrodenleitung aus körperfremdem Material zu keiner Abstoßungsreaktion führt.

Aus Sicht des Fachmannes oder Mediziners ist an diesem natürlichen Verwachsen jedoch nachteilig, dass die Explantation einer Elektrodenleitung der vorgenannten Art sehr schwierig ist, und auch manchmal unmöglich. Nicht selten sind diese Explantationen lebensgefährlich für den Patienten.
Explantationen sind beispielsweise dann notwendig, wenn die implantierte Elektrodenleitung defekt ist, also keine zuverlässige Stimulation oder Messwertaufnahme mehr ermöglichen. Dies kann zu schwerwiegenden und lebensgefährlichen Fehlfunktionen des elektrischen Implantats führen. Aufgrund der Komplexität und Lebensgefahr einer Explantation entschlossen sich in früherer Zeit viele Ärzte, anstatt defekte Elektroden zu explantieren, diese einfach funktionslos im Körper zu belassen und neue funktionsfähige Elektrodenleitungen in das Herz zu führen.
Es gibt jedoch auch Indikationen, bei denen ein Arzt keine Wahlmöglichkeiten hat und die Elektrodenleitung in jedem Fall zu explantieren hat. Als Beispiele hierfür werden Infektionen der so genannten Schrittmachertasche - so wird der Implantationsort des Implantats genannt - oder eine Sepsis entlang der Elektrodenleitung genannt. In diesen Fällen muss der Infektionsherd schnellstens entfernt werden, was zu einer unweigerlichen Explantation der implantierten Elektrodenleitung führt.

Um in diesen Fällen die Explantation der implantierten Elektrodenleitung zu erleichtern oder zu ermöglichen, sind verschiedene Hilfsmittel bekannt. Insbesondere sind Schneidwerkzeuge - so genannte Cutting-Sheath - bekannt. Dabei handelt es sich in der Regel um einen schlauch- oder rohrförmigen Körper mit einem Lumen und einem proximalen und einem distalen Ende, wobei dessen distales Ende eine Ablöseeinrichtung aufweist. Dieses Cutting-Sheath wird zur Explantation über die implantierte Elektrodenleitung geführt und zu deren distalem Ende vorgeschoben. Die Ablöseeinrichtung wirkt dann lösend auf das Gewebe, welches um die Elektrodenleitung verwachsen ist. Als Ablöseeinrichtungen sind aus dem Stand der Technik Schneiden bekannt, welche mittels einer in distale Richtung weisende Klinge das Gewebe von der Elektrodenleitung ablöst oder "abschabt". Ein Cutting-Sheath mit einer solchen Ablöseeinrichtung ist technisch einfach, aber stellt nicht das wirkungsvollste Mittel dar, um Gewebe von einer eingewachsenen Elektrodenleitung zu entfernen.
Ebenfalls aus dem Stand der Technik sind Ablöseeinrichtungen, welche auf Hochfrequenz- oder Lasertechnologie basieren, bekannt. Solche Ablöseeinrichtungen sind technisch so aufwändig, dass die Fehlerquote bei der Bedienung sehr hoch ist. Ein weiterer Grund für diese hohe Fehlerquote ist, dass die Bedienung nicht leicht ist und dem Arzt durch diese "aktive" Schneidetechnik jegliches Gefühl verloren geht.

Für alle vorgenannten Lösungen muss zudem eine Gegenkraft aufgebracht werden, welche durch Verankerung eines so genannten Locking-Stylets am distalen Ende der implantierten Elektrodenleitung aufgebracht wird. Zur Aufbringung der Gegenkraft wird das Locking-Stylet in das zentral verlaufende Lumen der implantierten Elektrodenleitung eingeführt, bis zum distalen Ende der Elektrodenleitung vorgeschoben und dort so verklemmt, dass sich die Elektrodenleitung nicht mehr bezüglich des Locking-Stylets bewegen kann. Anschließend wird das Stylet extern des Körpers fixiert und das Cutting-Sheath mit der Ablöseeinrichtung relativ zur Elektrodenleitung mit Locking-Stylet verschoben. Die implantierte Elektrodenleltung ist also fixiert und wirkt damit entgegen der in Richtung distales Ende der implantierten Elektrodenleitung gerichteten Schneidkraft. Ein Beispiel für ein solches Locking-Stylet ist in der US 6,358,256 B1 aufgeführt.

Locking-Stylets haben mehrere Nachteile. Zum einen ist die Nutzung eines solchen Stylets immer vom Vorhandensein eines zentral verlaufenden Lumens abhängig. Gerade neuere Elektrodenleitungen zur Anwendung im CS oder Nervenelektroden haben vielfach kein zentral verlaufendes Lumen mehr. Zum anderen ist es vorteilhaft, die Ansatzstelle der Gegenkraft immer in der Nähe der relativ zur Elektrodenleitung verschiebbaren Ablöseeinrichtung zu positionieren. Bei den Locking-Stylet ist jedoch ein Lösen der Verklemmung und ein Repositionierung bezüglich der Ablöseeinrichtung am Cutting-Sheath nicht möglich. Dies führt zu großen Problemen bei der Entfernung von Gewebe im mehr proximalen Bereich der Elektrodenleitung, da die Elektrodenleitung dort sehr weich und flexibel ist und die Gegenkraft somit nicht unmittelbar übertragen werden kann. Des Weiteren ist es wichtig, dass die Elektrodenleitung im äußeren Umfang keine Veränderung erfährt, um das Ablösen der Elektrodenleitung nur mit der notwendigen Schädigung des Gewebes durchzuführen.

Eine Weiterentwicklung des Locking-Stylet stellt gemäß des Standes der Technik ein "Locking-Sheath" dar, welches ebenfalls dazu dient, eine der Schneidkraft entgegenwirkende Kraft aufzubringen. Dieses Locking-Sheath umfasst in der Regel einen schlauch- oder rohrförmigen Körper mit einem Lumen entlang einer Längsachse und einem distalen und proximalen Ende. Am distalen Ende weist das Locking-Sheath eine Klemmvorrichtung auf, deren Klemmrichtung radial in Richtung Längsachse wirkt. Das Locking-Sheath wird zur Explantation über die implantierte Elektrodenleitung geführt und zur Verwachsung vorgeschoben. Dort wird die Klemmvorrichtung ausgelöst, das heißt, sie wirkt in Richtung Elektrodenleitung und klemmt die Elektrodenleitung so, dass sich die Elektrodenleitung nicht mehr bezüglich des Locking-Sheath bewegen kann. Anschließend wird das Locking Sheath extern des Körpers fixiert und das Cutting-Sheath mit der Ablöseeinrichtung relativ zum Locking-Sheath mit Elektrodenleitung verschoben. Die implantierte Elektrodenleitung ist also fixiert und wirkt damit entgegen der in Richtung distales Ende der implantierten Elektrodenleitung gerichteten Schneidkraft. Ein Beispiel für ein solches Locking-Sheath ist in der US 4,576,162 aufgeführt.
Nachteilig an der dort beschriebenen Lösung ist, dass das distale Ende des zwischen Cutting-Sheath und Elektrodenleitung liegenden Locking-Sheath zur Entlastung der Klemmvorrichtung aus dem distalen Ende des Cutting-Sheath herausgeschoben werden muss. Das ist unzweckmäßig, da sich am Locking-Sheath keine Schneide befindet. Das heißt, es kann nur in begrenztem Umfang in die Verwachsung eindringen, was ein mehrmaliges Nachjustieren während der Prozedur bedeutet. Zudem ist die Elektrodenleitung während des Schneidvorgangs immer fixiert, was unter Umständen nicht wünschenswert ist.

Gemäß einer weiteren Ausgestaltung des Locking-Sheath kann am distalen Ende auch eine Ablöseeinrichtung angebracht sein. Ein Beispiel dafür zeigt das Dokument US 2010/0198229. Dort wird die Klemmung am proximalen Ende mittels Hydraulik ausgeführt.

Die hydraulische Klemmung ist jedoch aufwändig zu realisieren und sie ist schwer manuell zu handhaben.

Die US 5,697,936 offenbart in Abb. 5 eine Explantationsvorrichtung zur Explantation implantierter Elektrodenleitungen, umfassend ein Locking-Sheath mit einer Klemmvorrichtung und ein Cutting-Sheath mit einer Ablöseeinrichtung.

Aufgabe der vorliegenden Patentanmeldung ist es daher, eine technisch einfache Explantationsvorrichtung für implantierte Elektrodenleitungen, welche einfach zu bedienen ist und welche verbesserte Fixierungsmöglichkeiten bietet, sowie ein Verfahren zu deren Anwendung bereit zu stellen.

Die Aufgabe wird durch eine Vorrichtung zur Explantation implantierter Elektrodenleitungen mit einem distalen und einem proximalen Ende gemäß Anspruch 1 gelöst. Die abhängigen Ansprüche beschreiben weitere Ausgestaltungsformen.

Die Explantationsvorrichtung umfasst einen Locking-Sheath zur lösbaren Befestigung an einer implantierten Elektrodenleitung mit einem distalen und proximalen Ende, das weiter einen schlauch- oder rohrförmigen Körper mit einem Lumen entlang einer Längsachse, welches sowohl am distalen als auch am proximalen Ende eine Öffnung aufweist, und mindestens eine Klemmvorrichtung am oder in der Nähe des distalen Endes umfasst welche aus einem Ruhezustand in einen gespannten Zustand und wieder zurück in den Ruhezustand überführbar ist. Des Weiteren umfasst die Explantationsvorrichtung einen Cutting-Sheath zur Entfernung von verwachsenem Gewebe mit einem proximalen und einem distalen Ende, das ebenfalls einen schlauch- oder rohrförmigen Körper mit einem Lumen entlang einer Längsachse umfasst, welches sowohl am proximalen als auch am distalen Ende eine Öffnung aufweist, und eine Ablöseeinrichtung am oder in der Nähe des distales Endes, wobei das Cutting-Sheath entlang der Längsachse frei in Bezug zum Locking-Sheath beweglich ist. Die Explantationsvorrichtung zeichnet sich dadurch aus, dass das Cutting-Sheath mindestens eine Aufnahme für die Klemmvorrichtung des Locking-Sheath umfasst, welche so gestaltet ist, dass die Klemmvorrichtung in ihrem Ruhezustand darin aufgenommen ist.

Dieser Lösung liegt die Erkenntnis zugrunde, dass es sinnvoll ist, eine universelle, an allen möglichen Elektrodenleitungen anwendbare Extraktionsvorrichtung zu haben, also auch für Elektrodenleitungen, welche nicht über ein zentral verlaufendes Lumen verfügen. Ebenso hat sich die Erkenntnis durchgesetzt, dass es sehr wichtig ist, eine technisch einfache Vorrichtung zu haben, welche während der Nutzung wenig fehlerhafte Handhabung zulässt und aufgrund der technischen Einfachheit wenige technische Defekte hervorruft.

Durch die Lage der aufgebrachten Gegenkraft direkt an der Ablösestelle des Gewebes wird die Wirksamkeit des Extraktionsvorganges erheblich verbessert.
Somit handelt es sich bei der obigen Lösung um eine hoch wirksame Extraktionsvorrichtung, welche die Extraktion von Elektrodenleitungen vereinfacht und weiterentwickelt.

Im gespannten Zustand einer Ausgestaltung der obigen Lösung fixiert die mindestens eine Klemmvorrichtung das Locking-Sheath an der Elektrodenleitung und löst im Ruhezustand die Fixierung zur Elektrodenleitung, wobei die Überführung vom Ruhezustand in den gespannten Zustand und vom gespannten Zustand in den Ruhezustand durch die Verschiebung des Cutting-Sheath und damit der mindestens einen Aufnahme entlang der Längsachse in Bezug zum Locking-Sheath erfolgt, so dass sich die mindestens eine Klemmvorrichtung aus der oder in die mindestens eine Aufnahme bewegt. Dadurch wird die Handhabung noch weiter vereinfacht und ein versehentliches Lösen der Elektrodenleitung während des Ablösevorganges wird vermieden.

Vorzugsweise ist die Klemmvorrichtung der Explantationsvorrichtung aus einem Ruhezustand in einen gespannten Zustand und/oder zurück in den Ruhezustand durch Federkraft überführbar ist. Alternativ sind andere Mechanismen vorstellbar, mit Hilfe derer die Klemmvorrichtung vom Ruhezustand in den gespannten Zustand und/oder vom gespannten Zustand in den Ruhezustand überführbar ist.

Vorteilig ist das Locking-Sheath mit dem Cutting-Sheath entlang der Elektrodenleitung führbar, wenn sich die mindestens eine Klemmvorrichtung in der mindestens einen Aufnahme befindet. Möglich ist auch, dass die gemeinsame Führung von Locking- und Cutting-Sheath lediglich temporär erfolgt, also das Cutting-Sheath nach dem Locking-Sheath in den Körper verbracht wird.
Dazu kann in einer Ausführung die mindestens eine Klemmvorrichtung und die mindestens eine Aufnahme je mindestens eine radiale Erstreckungskomponente umfassen. In diesem Fall ist die radiale Erstreckungskomponente eine im Wesentlichen von der Längsachse der Explantationsvorrichtung weg weisende Richtung.
Wenn sich also die Klemmvorrichtung im Ruhezustand befindet - sie übt dann keine Klemmkraft auf die Elektrodenleitung aus, kann die Explantationsvorrichtung unabhängig von der Elektrodenleitung verschoben werden. In diesem Zustand kann sie also entweder in distale Richtung vorgeschoben werden oder aber über der Elektrodenleitung herausgezogen werden. Das erleichtert weiter die Arbeit des Operateurs, der sich damit ganz auf die gefährliche Explantation konzentrieren kann und von der komplizierten Handhabung der Explantationsvorrichtung befreit ist. Befindet sich die Klemmvorrichtung jedoch nicht in der Aufnahme und ist im gespannten Zustand, so ist ein Verschieben der Elektrodenleitung nicht mehr möglich. In diesem Zustand wirkt eine Gegenkraft und das Ablösen von schweren Verwachsungen ist möglich. In diesem Zustand kann die Elektrodenleitung auch "gezogen" werden, das heißt, sie kann mittels der Explantationsvorrichtung und nach komplettem Entfernen des Gewebes aus dem Körper explantiert werden.

Dann ist es auch bevorzugt vorstellbar, dass die mindestens eine Klemmvorrichtung eine Spannzange ist, welche mindestens eine, vorzugsweise vier, in Richtung der Längsachse erstreckende Spannlaschen umfasst, wobei jede Spannlasche einen ersten Abschnitt, der an einem ersten Ende am Locking-Sheath angebracht ist, und einen zweiten Abschnitt mit einem radial in Richtung Längsachse ragendes Ende aufweist. Eine mögliche zusätzliche Ausgestaltung sieht vor, dass sich die Spannlaschen parallel zueinander in Richtung der Längsachse erstrecken.
Besonders bevorzugt weist der erste Abschnitt der Spannlaschen im Ruhezustand eine von seinem ersten Ende zu einem zweiten Ende hin radial von der Längsachse weg gerichtete Erstreckungskomponente, und der zweite Abschnitt, der am zweiten Ende des ersten Abschnittes angebracht ist, eine zum nach innen ragenden Ende hin radial zur Längsachse hin gerichtete Erstreckungskomponente auf. Dadurch erhält die Klemmvorrichtung im Ruhezustand eine radial nach außen gerichtete Auswölbung, in der sie sich in der ebenfalls nach außen gewölbten Aufnahme befindet.
Verschiebt man nun das Cutting-Sheath in Bezug zum Locking-Sheath, so wird die Klemmvorrichtung in den gespannten Zustand überführt, so dass das innere Ende weiter bevorzugt in das Lumen des Locking-Sheath hineinragt. Dadurch kann die Klemmvorrichtung eine Klemmkraft auf die Elektrodenleitung ausüben. Durch diese Ausgestaltung ist es dem Operateur also möglich, durch reine passive Betätigung und ohne zusätzliche Hilfsmittel die Klemmung durchzuführen, ohne dabei die Konzentration von der eigentlichen Ablösung der Elektrodenleitung zu lassen.
Das nach innen ragende Ende ist so geformt, dass es ein sicheres Fixieren ohne Verrutschmöglichkeit bietet. Deshalb kann es zum nach innen ragenden Ende hin abgerundet, eckig angeschliffen, als Schneide angeschliffen aufgeraut und/oder mit einer rutschfesten Beschichtung versehen sein.

Damit die Betätigung dieser Klemmung gemäß der soeben genannten Ausführung leicht durchführbar ist, umfasst die mindestens eine Aufnahme einen proximalen Wandungsabschnitt und einen distalen Wandungsabschnitt mit je einer Erstreckungskomponente in radialer Richtung, wobei in Richtung des distalen Endes des Cutting-Sheath hin der proximale Wandungsabschnitt eine radial von der Längsachse weg gerichtete Richtungskomponente aufweist und der distale Wandungsabschnitt eine radial zur Längsachse hin gerichtete Richtungskomponente aufweist. So wird eine leichtgängige Fixierung und Lösung ermöglicht, was wiederum ein unkontrolliertes Verrutschen des Cutting-Sheath und damit ungewollte Verletzungen verhindert.
Eine weitere Erleichterung stellt bevorzugt dar, wenn mindestens einer der beiden Wandungsabschnitte der Aufnahme, vorzugsweise der proximale Wandungsabschnitt, je eine Erstreckungsrichtung parallel zu den ersten bzw. zu den zweiten Abschnitten der mindestens einen Spannlasche der mindestens einen Klemmvorrichtung aufweisen.

In einer speziellen Ausführung ist das Locking-Sheath der Explantationsvorrichtung besonders bevorzugt radial innerhalb des Cutting-Sheath in dessen Lumen beweglich geführt und die implantierte Elektrodenleitung radial innerhalb des Locking-Sheath in dessen Lumen einführbar, so dass die Klemmvorrichtung durch Verschieben aus der Aufnahme in das im Innendurchmesser an den Körper des Locking-Sheath angepasste Lumen in den gespannten Zustand überführt wird und dadurch die implantierte Elektrodenleitung fixiert.

Des Weiteren wird die Aufgabe durch ein Verfahren zur Explantation implantierter Elektrodenleitungen mittels einer soeben beschriebenen Explantationsvorrichtung gelöst, welches die folgenden auszuführenden Schritte umfasst:
a) Einführen des proximalen Endes der implantierten Elektrodenleitung in die distalen Lumenöffnungen des Cutting-Sheath und des Locking-Sheath der Explantationsvorrichtung, bei der sich die Klemmvorrichtung des Locking-Sheath im Ruhezustand in der Aufnahme des Cutting-Sheath befindet und damit der Locking-Sheath (auch temporär) mit dem Cutting-Sheath geführt wird;
b) Vorschieben der Explantationsvorrichtung in Richtung distales Ende der implantierten Elektrodenleitung und dabei Ablösen von eventuell vorhandenen leicht anhaftenden Gewebeverwachsungen an der Elektrodenleitung;
c) bei Erreichen von starken Verwachsungen oder hohem Widerstand beim Vorschieben der Explantationsvorrichtung in Richtung distales Ende der Elektrodenleitung: Fixieren des Locking-Sheath zur Verhinderung einer Bewegung in Bezug zur implantierten Elektrodenleitung;
d) Verschieben des Cutting-Sheath in distaler Richtung in Bezug zum Locking-Sheath, so dass sich die Klemmvorrichtung in proximaler Richtung aus der Aufnahme bewegt, wobei sich das Locking-Sheath fest mit der implantierten Elektrodenleitung verklemmt und wobei die Ablöseeinrichtung des Cutting-Sheath die starken Verwachsungen von der Elektrodenleitung ablöst;
e) Verschieben des Cutting-Sheath in proximaler Richtung in Bezug zum Locking-Sheath, so dass sich die Klemmvorrichtung in distale Richtung in die Aufnahme bewegt, wobei sich die Fixierung des Locking-Sheath von der implantierten Elektrodenleitung löst; und
f) Gegebenenfalls Wiederholen der Schritte c) bis e), bis die implantierte Elektrodenleitung frei von Verwachsungen ist und zusammen mit der Explantationsvorrichtung aus dem Körper entfernt werden kann.

Der Gegenstand der Patentanmeldung wird nun anhand der beiliegenden Fig. beschrieben. Dabei zeigt:
- Fig. 1A: einen Längsschnitt eines Locking-Sheath gemäß einer ersten Ausführung dieser Patentanmeldung
- Fig. 1B: eine perspektivische Darstellung der Klemmvorrichtung des Locking-Sheath im Ruhezustand gemäß der ersten Ausführung
- Fig. 1C: eine perspektivische Darstellung der Klemmvorrichtung des Locking-Sheath im gespannten Zustand gemäß der ersten Ausführung
- Fig. 2A: Längsschnitt eines Locking-Sheath gemäß einer zweiten Ausführung dieser Patentanmeldung
- Fig. 2B: eine perspektivische Darstellung der Klemmvorrichtung des Locking-Sheath im Ruhezustand gemäß der zweiten Ausführung
- Fig. 2C: eine perspektivische Darstellung der Klemmvorrichtung des Locking-Sheath im gespannten Zustand gemäß der zweiten Ausführung
- Fig. 3A: ein Cutting-Sheath gemäß einer Ausführung dieser Patentanmeldung
- Fig. 3B: einen Längsschnitt des Cutting-Sheath aus Fig. 3A
- Fig. 4A: einen Längsschnitt durch die Explantationsvorrichtung im Ruhezustand in der ersten Ausführungsform
- Fig. 4B: einen Längsschnitt durch die Explantationsvorrichtung im gespannten Zustand in der ersten Ausführungsform
- Fig. 5A: einen Längsschnitt durch die Explantationsvorrichtung im Ruhezustand in der zweiten Ausführungsform
- Fig. 5B: einen Längsschnitt durch die Explantationsvorrichtung im gespannten Zustand in der zweiten Ausführungsform
- Fig. 6A: eine perspektivische Ansicht des proximalen Endes der Explantationvorrichtung, bei der die Spannzange vom Ruhezustand in den gespannten Zustand verbracht wird
- Fig. 6B: eine perspektivische Ansicht des proximalen Endes der Explantationvorrichtung, bei der die Spannzange vom gespannten Zustand in den Ruhezustand verbracht wird

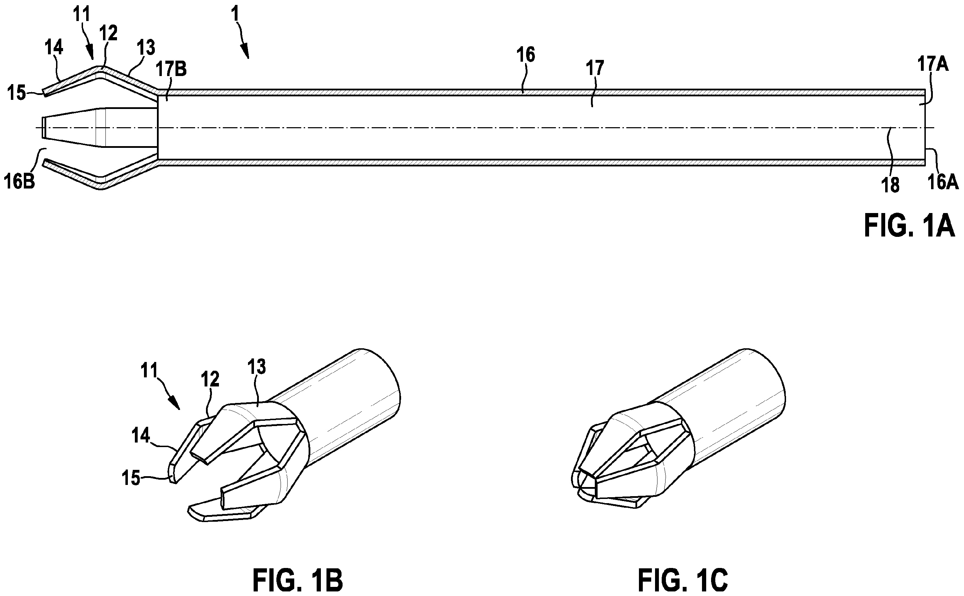

Fig. 1A zeigt ein Locking-Sheath 1 gemäß einer ersten Ausführung der Patentanmeldung. Das Locking-Sheath 1 umfasst einen rohr- oder schlauchförmigen Körper 16 mit einem proximalen Ende 16A und einem distalen Ende 16B, welcher sich entlang einer Längsachse 18 erstreckt und welcher ein Lumen 17 umschließt. Das Lumen 17 hat sowohl am proximalen Ende als auch am distalen Ende eine Öffnung 17A und 17B. Sowohl das Lumen 17 als auch seine Öffnungen 17A und 17B sind so ausgestaltet, dass eine Elektrodenleitung darin einführbar und dort auch verschieblich gelagert ist.
Am distalen Ende des Locking-Sheath 1 befindet sich in Verlängerung zur distalen Öffnung 17B des Lumens 17 eine Klemmvorrichtung 11, welche in Fig. 1A im Ruhezustand dargestellt ist. Die Klemmvorrichtung 11 dient dazu, eine im Lumen 17 eingeführte verschieblich gelagerte Elektrodenleitung temporär zu fixieren. Im dargestellten Ruhezustand der Klemmvorrichtung 11 ist es möglich, die Elektrodenleitung in das Lumen 17 einzuführen bzw. das Locking-Sheath 1 über die Elektrodenleitung zu schieben.

Fig 1B und 1C zeigen in perspektivischer Darstellung die Klemmvorrichtung 11 eines Locking-Sheathes 1 im Ruhezustand (Fig. 1B) und im gespannten Zustand (Fig. 1C). Die Klemmvorrichtung ist in der Regel eine Spannzange, welche mindestens eine, vorzugsweise vier, in Richtung der Längsachse 18 erstreckende Spannlaschen 12 umfasst. In diesem Ausführungsbeispiel hat jede Spannlasche einen ersten Abschnitt 13, der an seinem ersten Ende am schlauchförmigen Körper 16 angebracht ist und von seinem ersten Ende zu einem zweiten Ende hin eine radial von der Längsachse 18 weg gerichtete Erstreckungskomponente aufweist. Ein zweiter Abschnitt 14, der vom ersten Abschnitt in distaler Richtung weg ragt und am zweiten Ende des ersten Abschnittes angebracht ist, weist eine radial zur Längsachse 18 gerichtete Erstreckungskomponente und ein radial in Richtung Längsachse 18 ragendes Ende 15 auf. Dieses nach innen ragende Ende 15 ist im Ruhezustand so positioniert, dass es nicht in das Innere des Lumens 17 bzw. in den Raum in distaler Verlängerung des Lumens ragt. Somit kann im Ruhezustand keine Klemmwirkung auf eine im Lumen verschiebbar gelagerte Elektrodenleitung ausgeübt werden.
Das nach innen ragende Ende 15 kann zum dem Ende hin, welches in den Klemmeingriff mit der Elektrodenleitung gebracht werden kann, abgerundet, eckig angeschliffen, als Schneide angeschliffen aufgeraut und/oder mit einer rutschfesten Beschichtung versehen sein.

Das Prinzip der Klemmvorrichtung 11 wird nun anhand einer der mehreren Spannlaschen 12 beschrieben. Es gilt selbstverständlich für alle Spannlaschen 12 einer Klemmvorrichtung 11. Wird ein in Richtung Längsachse 18 gerichteter radialer Druck auf das radial von der Längsachse 18 weg ragende zweite Ende des ersten Abschnittes 13 ausgeübt, das heißt, die Klemmvorrichtung 11 wird vom in Fig. 1A und 1B dargestellten zum in Fig. 1C dargestellten gespannten Zustand überführt, wird das in Richtung Längsachse ragende Ende 15 des zweiten Abschnittes 14 der Spannlasche 12 nach innen gedrückt, so dass das Ende in das Innere des Lumens 17 bzw. in den Raum in distaler Verlängerung des Lumens ragt. In diesem Zustand gelangt das Ende 15 in Eingriff mit der Elektrodenleitung. Dabei wird die vorher im Locking-Sheath 1 eingeführte und verschiebbar gelagerte Elektrodenleitung relativ zum Locking-Sheath 1 fixiert. Wird der genannte Druck auf das zweite Ende des ersten Abschnittes 13 wieder entfernt, so lenkt sich die Spannlasche 12 aufgrund der Federkraft des Materials zurück in den Ruhezustand und der Eingriff in die Elektrodenleitung wird aufgehoben. Auf diese Weise kann die im Lumen 17 des Locking-Sheath 1 befindliche Elektrodenleitung gefasst und wieder freigegeben werden. Dieses Prinzip kann auch auf andere Implantate angewandt werden, beispielsweise auf einen leitungsgebundenen implantierbaren Sensor oder einen leitungslosen Herzschrittmacher ("leadless pacer").
Die in Fig. 1A bis 1C gezeigte Ausführung des Locking-Sheath 1 hat eine Klemmvorrichtung 11, welche so gestaltet ist, dass die Spannlaschen 12 in Richtung des distalen Endes 16B gerichtet sind. Diese Ausführungsform ist einfach herzustellen und hat im gespannten Zustand eine etwas weniger schädigende Klemmwirkung auf die Elektrodenleitung, welche oberhalb einer bestimmten Zugkraft durch die Klemmvorrichtung 11 hindurch gleiten kann.

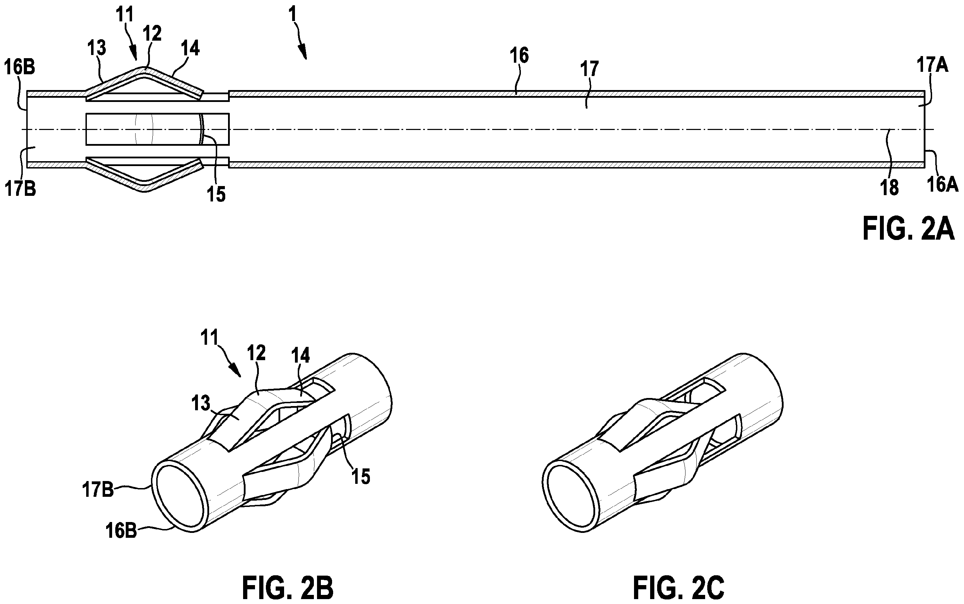

Eine weitere Ausführung eines Locking-Sheath ist den Fig. 2A bis 2C zu entnehmen. Die dort gezeigten Bezugszeichen kennzeichnen dieselben Merkmale wie die in Fig. 1A bis 1C gezeigten Bezugszeichen. Im Unterschied zu der in Fig. 1A bis 1C gezeigten Ausführung ist die Klemmvorrichtung 11 des Locking-Sheath 1 nicht in Verlängerung der distalen Öffnung 17B des Lumens 17 angebracht, sondern in proximaler Richtung versetzt auf dem schlauchförmigen Körper 16 des Locking-Sheath 1. Das distale Ende 16B des Körpers 16 fällt also mit der distalen Öffnung 17B des Lumens zusammen. Durch diese in proximale Richtung versetzte Anordnung ist auch eine andere Konfiguration der Spannlaschen 12 möglich. So ist es gemäß dieser Ausführung möglich, dass die Spannlaschen 12 nicht am distalen Ende 17B des Lumens 17 angebracht ist, sondern in proximaler Richtung vor dem distalen Ende 17B des Lumens 17. Die übrige Formung der Spannlaschen mit einem ersten und zweiten Abschnitt 13 und 14 sowie die Klemmwirkung sind identisch zu den in Fig. 1A bis Fig. 1C beschriebenen, wobei aber der erste Abschnitt 13 am ersten Ende in Richtung distales Ende 16B am Körper 16 des Locking-Sheath 1 angebracht ist.
Diese Ausführungsform ist etwas komplizierter herzustellen, die Klemmwirkung ist aber in Zugrichtung (in Richtung proximales Ende 16A) wesentlich wirkungsvoller, da sich die das in Richtung Längsachse ragende Ende 15 der Spannlaschen 12 in die Isolation der Elektrodenleitung hinein schneiden und so eine zusätzliche Gegenkraft erzeugen.
Die Federkraft der gebogenen Spannlaschen 12 bestimmt die Kraft, mit der die Elektrodenleitung oder das Implantat gefasst wird.

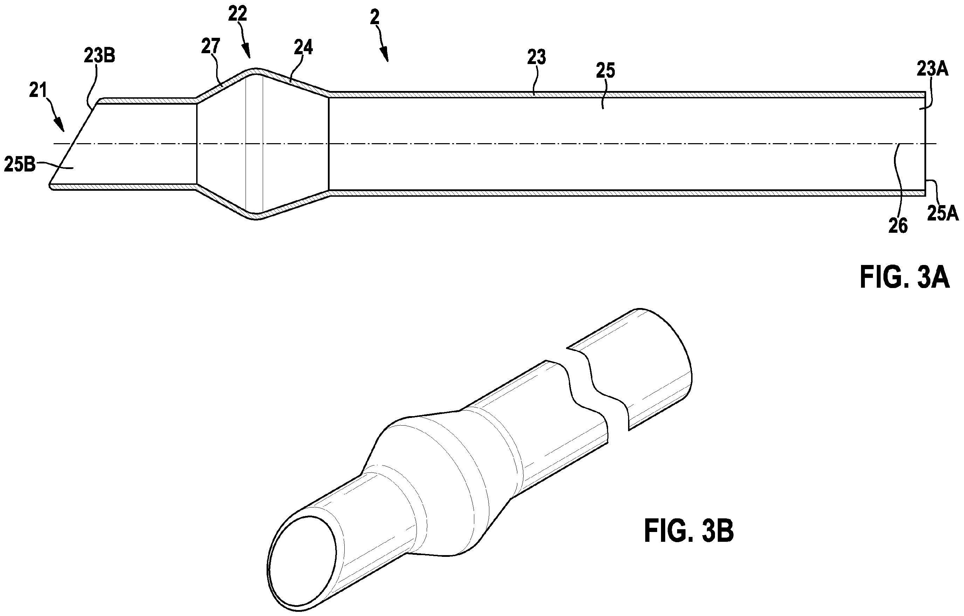

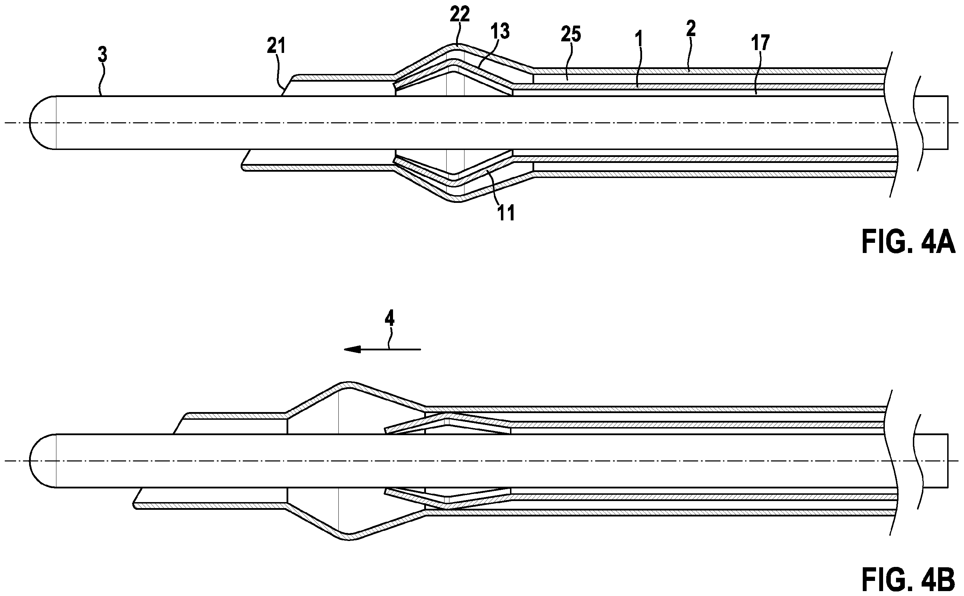

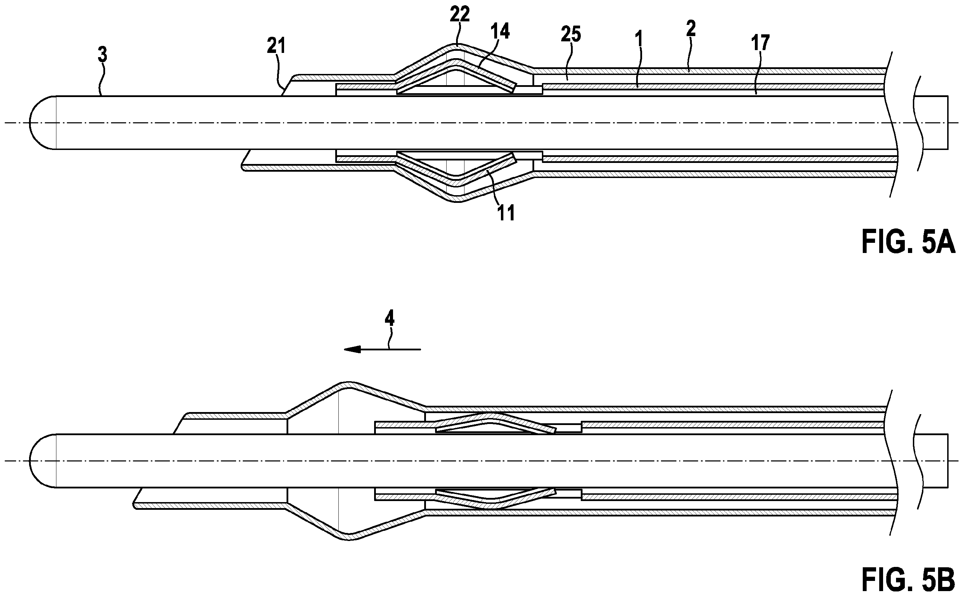

Fig. 3A und 3B zeigen ein Cutting-Sheath, mit dessen Hilfe sowohl das Gewebe von der Elektrodenleitung abgelöst als auch die Klemmvorrichtung 11 des Locking-Sheath bedient bzw. ausgelost wird. Das Cutting-Sheath 2 zur Entfernung von verwachsenem Gewebe umfasst einen schlauch- oder rohrförmigen Körper 23 mit einem proximalen und einem distalen Ende 23A und 23B sowie ein Lumen 25 entlang einer Längsachse 26 mit einer Öffnung 25A und 25B sowohl am proximalen als auch am distalen Ende. Eine Ablöseeinrichtung 21 am oder in der Nähe des distalen Endes 23B ist dazu ausgebildet, das verwachsene Gewebe von der Elektrodenleitung abzulösen. In der Regel handelt es sich bei dieser Ablöseeinrichtung um eine Klinge, möglich sind aber auch andere eingangs genannte Einrichtungen.
In der bevorzugten Ausführung ist das Cutting-Sheath 2 so ausgestaltet und dimensioniert, dass das Locking-Sheath 1 in das Lumen 25 eingeführt werden kann und dort verschieblich gelagert ist. Allerdings ist der Innendurchmesser des Lumens 25 an den Außendurchmesser des Körpers 16 des Locking-Sheath 1 angepasst, was bedeutet, dass der Innendurchmesser 25 wesentlich geringer ist als der äußere Durchmesser der Klemmvorrichtung 11 ist, welcher durch das zweite Ende des ersten Abschnitt 13, der von seinem ersten Ende zu seinem zweiten Ende hin eine radial von der Längsachse 18 weg gerichtet ist, gebildet wird. Somit wäre also die Klemmvorrichtung 11 immer im gespannten Zustand, wenn das Locking-Sheath in ein Cutting-Sheath herkömmlicher Art eingeführt wäre. Damit eine Aktivierung und ein Gebrauch möglich sind, umfasst das Cutting-Sheath 2 im distalen Abschnitt eine Aufnahme 22 in Form einer Aussparung. Die Aussparung hat einen größeren Innendurchmesser als der Körper 23 des Cutting-Sheath. In der Regel entspricht der Innendurchmesser der Aufnahme 22 im Wesentlichen mindestens dem Außendurchmesser der Klemmvorrichtung 11. Bevorzugt umfasst die Aufnahme 22 einen proximalen und einen distalen Wandungsabschnitt 24 und 27. Beide Wandungsabschnitte sind in dieser Ausführung geneigt von der Längsachse 26 weg oder zur Längsachse 26 hin geneigt, sie weisen also neben einer radial von der Längsachse wegführenden Erstreckungskomponente auch eine Erstreckungskomponente parallel zur Längsachse auf.
Im Ruhezustand der Spannlasche 12 befindet sich die Klemmvorrichtung 11 in der Aufnahme 22 und übt keine Klemmwirkung auf die Elektrodenleitung 3 aus, die sich im Lumen des Locking-Sheath eingeführt befindet und dort verschieblich gelagert ist. Dieser Zustand wird in Fig 4A und 5A dargestellt. In diesem Zustand kann der Anwender die Vorrichtung zur Extraktion bestehend aus Cutting- und Locking-Sheath so handhaben wie ein bekanntes, einzelnes Sheath, d.h. Vorrichtung lässt sich relativ zur Elektrodenleitung 3 verschieben, um beispielsweise leicht anhaftendes Gewebe mittels der Ablöseeinrichtung 21 ohne Gegenkraft oder nur mittels der Gegenkraft durch ein herkömmliches Locking Stylet abzulösen. Des Weiteren kann in dieser Position des Locking-Sheath 1 die Vorrichtung bis zur gewünschten Stelle über die Elektrodenleitung in distale Richtung geschoben werden, an der die Gegenkraft angelegt werden soll.

Die Handhabung und Wirkweise der Vorrichtung wird ausgehend von der soeben beschriebenen Erläuterung und ausgehend von den Fig. 4A und 5A nun anhand der folgenden Beschreibung und der Fig. 4B und 5B beschrieben. In letzteren Fig. ist der Zustand dargestellt, in dem die Spannlaschen 12 der Klemmvorrichtung 11 im gespannten Zustand sind und eine Klemmwirkung auf die Elektrodenleitung 3 ausübt. Die Klemmwirkung wird durch die Verschiebung des Cutting-Sheath 2 gegenüber dem Locking-Sheath 1 beispielsweise in Pfeilrichtung 4 in distale Richtung bzw. durch Verschiebung des Locking-Sheath 1 gegenüber dem Cutting-Sheath 2 in proximale Richtung hergestellt. Dabei gleiten die Spannlaschen 12 der Klemmvorrichtung 11 aus der Aussparung der Aufnahme 22 in das Lumen 25 des Körpers 23 - welches einen wesentlich geringeren Durchmesser aufweist als der äußere Durchmesser der Klemmvorrichtung 11, wodurch die radial nach innen ragenden Enden 15 der Spannlaschen 12 nach innen ausgelenkt und gegen die Außenhülle der Elektrodenleitung 3 gedrückt werden. Im ausgelenkten Zustand übt jede einzelne Spannlasche 12 der Klemmvorrichtung 11 eine Klemmwirkung auf die Elektrodenleitung 3 aus, wodurch diese so fixiert wird, dass sie gegenüber dem Locking-Sheath 1 nicht mehr verschieblich ist. Nach erfolgter Fixierung kann mittels der Abschälvorrichtung 21 des Cutting-Sheath 2 das Gewebe, in welches die Elektrodenleitung 3 eingewachsen ist, in distale Richtung abgeschält werden. Die Klemmvorrichtung 11 bleibt dabei immer innerhalb des Lumens 25.
Während einer Explantation kann der Klemmwirkung beliebig oft gelöst und wieder angelegt werden, da es notwendig ist, dass die Gegenkraft, welche durch die Klemmwirkung der Klemmvorrichtung 11 wirkt, möglichst in der Nähe der Abschälposition anliegt. Um ein leichtes Verschieben des Locking-Sheath 1 gegenüber dem Cutting-Sheath 2 vom Ruhezustand der Klemmvorrichtung 11 zum gespannten Zustand zu ermöglichen, sind die beiden Wandungen 24 (proximal) und 27 (distal) der Aufnahme 22 wie oben dargestellt von der Längsachse 26 weg oder zur Längsachse 26 hin geneigt. Besonders erleichtert wird die Überführung in den gespannten Zustand der Klemmvorrichtung 11, wenn sich zumindest die Wandung 24 parallel zum proximalen Abschnitt erstreckt. Im Fall der in Fig. 4A und Fig. 4B dargestellten Ausführung ist es vorteilhaft, wenn die Parallelität zwischen der proximalen Wandung 24 und dem ersten Abschnitt 13 der Spannlasche 12 existiert, und im Falle der in Fig. 5A und Fig. 5B dargestellten Ausführung existiert die Parallelität vorteilhafterweise zwischen der Wandung 24 und zweitem Abschnitt 14 der Spannlasche 12.

In allen Fig. sind die nach innen ragenden Enden 15 der Spannlaschen 12 ohne eine Form dargestellt, um das Prinzip zu zeigen. Jedoch kann die Form und Gestaltung der Spannlaschen angepasst sein, um die Wirkung zu verbessern. So ist es denkbar, die nach innen ragenden Enden 15 abgerundet, eckig angeschliffen, als Schneide angeschliffen oder anderweitig aufgeraut zu formen oder mit einer rutschfesten Beschichtung zu versehen.
Je nach Anforderung kann sowohl die Federwirkung als auch die Anzahl der Spannlaschen 12 variieren, um auf Elektrodenleitungen verschiedenen Durchmessers zu wirken.

Gemäß einer weiteren Ausbildung ist es möglich, die in Fig. 1A bis Fig. 1C gezeigte Ausführung mit der Ausführung der Fig. 2A bis 2C zu kombinieren und mehrere Klemmvorrichtungen 11 hintereinander und entlang des Locking-Sheath 1 anzuordnen. Dementsprechend weist das dazugehörige Cutting-Sheath 2 mehrere Aufnahmen 22 auf, in denen im Ruhezustand die Klemmvorrichtungen 11 ruhen. Zwischen zweien der Aufnahmen 22 kann derselbe Abstand sein wie zwischen den beiden zugeordneten Klemmvorrichtungen 11. Gegebenenfalls kann der Abstand jedoch auch unterschiedlich sein.
So ist es möglich, die Klemmwirkung bei verschiedenen Verschiebestrecken zu aktivieren, um so die Gegenkraft Zug um Zug aufzubringen bzw. zu variieren.

Sowohl das Locking-Sheath 1 jeglicher der genannten Ausführungen als auch das Cutting-Sheath 2 können aus Metallen, insbesondere medizinischen Edelstählen oder Kunststoffen (z.B. Teflon, PTFE, Polypropylen, Polyamid, PEABA, Polyurethan, Polyimid) oder aus einer Komposition aus verschiedenen Materialien und/oder verschiedenen Kunststoffen gefertigt sein.
Dabei kann auch vorgesehen sein, visuelle Marker vorzusehen, welche per bildgebendem Verfahren (Magnetresonanztomographie, Ultraschall, Röntgen) anzeigen, in welcher Position Locking- und Cutting-Sheath zueinander sind. So kann festgestellt werden, ob sich die Klemmvorrichtung im Ruhe- oder im gespannten Zustand befindet.

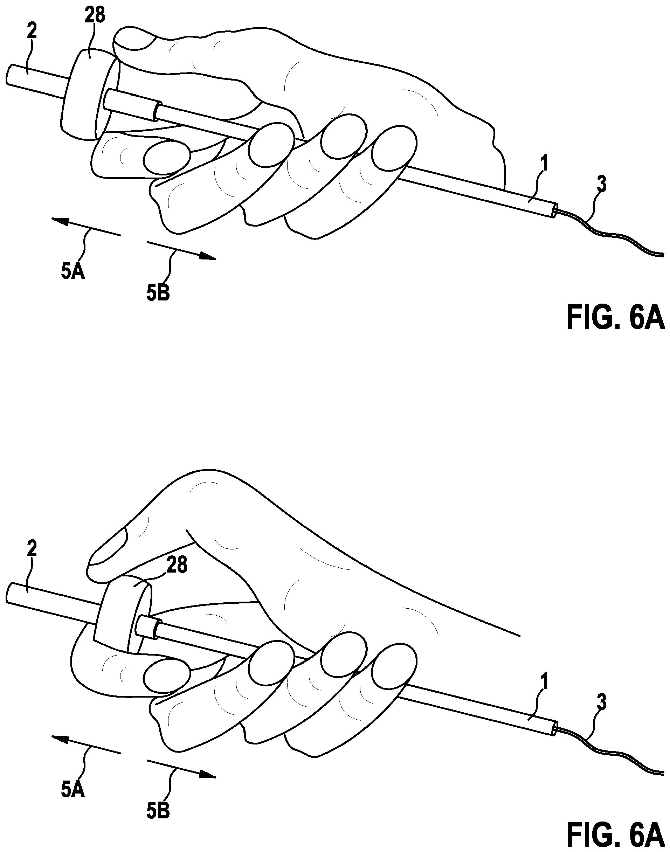

Fig. 6A und Fig. 6B zeigen das proximale Ende der Explantationsvorrichtung bestehend aus dem Locking-Sheath 1 und dem Cutting-Sheath 2 gemäß einem der genannten Ausführungen, welche sich in beiden Fig. im Gebrauchszustand einer vorteilhaften Ausführung in der Hand eines Operateurs oder Arztes befindet. In dieser Ausführung ist die Explantationsvorrichtung so gestaltet, dass sich das Locking-Sheath 1 zwischen der im Lumen 17 befindlich Elektrodenleitung 3 und dem Cutting-Sheath 2 befindet. Die Längen der beiden Sheath' 1 und 2 sind so gestaltet, dass das Locking-Sheath 1 aus dem Cutting-Sheath 2 proximal herausragt, vorzugsweise zwischen 10 und 20 cm, so dass beide Sheath' vom Operateur gleichzeitig in einer Hand gefasst und gegeneinander verschoben werden können (in Richtung der Pfeile 5A und 5B).
Vorteilhafterweise sind die Enden sowohl des Locking-Sheath 1 als auch des Cutting-Sheath 2 so gestaltet, dass das Greifen und gegeneinander Verschieben erleichtert ist. So kann vorgesehen sein, am proximalen Ende des Cutting-Sheath 2 eine Schiebevorrichtung 28 - beispielsweise in Form einer Schaftverdickung, eines Schiebegriffs, einer Schiebewulst oder eines Schiebeknopfs - anzubringen, welches ein Vor- und Zurückschieben mittels Daumen und Zeigefinger erleichtert, ohne dass die Position des Locking-Sheathes 1 verändert wird. Ergänzend dazu kann das proximale Ende des Locking-Sheath mit einer rutschhemmenden Einprägung (Aufrauhung) oder einer Beschichtung (beispielsweise Silikon) versehen sein, die das Festhalten mit der Hand während der Relativverschiebung des Cutting-Sheathes 2 ermöglicht (zum Anbringen einer Gegenkraft), aber auch ein Greifen, Ziehen oder Schieben des Cutting-Sheath mit Daumen und Zeigefinger relativ zum mit Mittel-, Ring- und Kleinfinger gehaltenen Locking-Sheath erleichtert.

Mit diesem vorteilhaften proximalen Aufbau der beiden Sheath' ist eine Bedienung mit einer Hand möglich. Bei Erreichen einer Verwachsung wird die Klemmvorrichtung 11 am Locking-Sheath 1 vom Ruhe- in den gespannten Zustand verbracht, in dem das Locking-Sheath 1 mittels Mittel-, Ring- und kleinem Finger festgehalten wird, während Daumen und Zeigefinger das Cutting-Sheath 2 mit Hilfe der Verdickung 28 am proximalen Ende in distale Richtung (Pfeilrichtung 5A) schieben (Fig. 6A). Ist die Verwachsung gelöst, wird die Klemmvorrichtung 11 am Locking-Sheath 1 der Explantationsvorrichtung vom gespannten in den Ruhezustand verbracht, indem das Cutting-Sheath 2 wieder zurückgezogen wird (in Richtung des Pfeiles 5B), bis die Klemmvorrichtung 11 in die Aufnahme 22 des Cutting-Sheath 2 gleitet und die Elektrodenleitung freigibt. Dazu wird das Locking-Sheath 1 beispielsweise wieder mit Mittel-, Ring- und kleinem Finger festgehalten, während Daumen und Zeigefinger das Cutting-Sheath 2 mit Hilfe der Verdickung 28 zurückziehen (Fig. 6B).

Des Weiteren kann sich beispielsweise im proximalen Endbereich des Locking-Sheath 1 eine optische Markierung befinden (beispielsweise in Form von Strichen, Bildern (Icons) oder Farben), mittels derer der Operateur erkennen kann, wie der Spannungszustand der Klemmzange 11 (Ruhe- oder gespannter Zustand) ist. Zur besseren Handhabung können am proximalen Ende der Sheath' ergonomisch geformte Griffe angeordnet sein, um das Gegeneinander-Verschieben einfach zu gestalten.

Weiter oben wird schrittweise die generelle Anwendung der Explantationsvorrichtung dargestellt. Jedoch umfasst der Ablauf in der Regel weitere Schritte und stellt sich wie folgt dar:
a) Freipräparation der Elektrodenleitung und Abschneiden des Steckers der Elektrodenleitung;
b) Einführen des proximalen Endes der implantierten Elektrodenleitung in die distalen Lumenöffnungen des Cutting-Sheath und des Locking-Sheath der Explantationsvorrichtung, bei der sich die Klemmvorrichtung des Locking-Sheath im Ruhezustand in der Aufnahme des Cutting-Sheath befindet und damit Locking-Sheath (auch temporär) mit dem Cutting-Sheath geführt wird;
c) Vorschieben der Explantationsvorrichtung in Richtung des distalen Endes der implantierten Elektrodenleitung und dabei Ablösen von leicht anhaftenden Gewebeverwachsungen an der Elektrodenleitung;
d) bei Erreichen von starken Verwachsungen oder hohem Widerstand beim Vorschieben der Explantationsvorrichtung in Richtung des distalen Endes der Elektrodenleitung: Fixieren des Locking-Sheath zur Verhinderung einer Bewegung in Bezug zur implantierten Elektrodenleitung;
e) Verschieben des Cutting-Sheath in distaler Richtung in Bezug zum Locking-Sheath, so dass sich die Klemmeinrichtung in proximaler Richtung aus der Aufnahme bewegt, wobei sich das Locking-Sheath fest mit der implantierten Elektrodenleitung verklemmt und wobei die Ablöseeinrichtung des Cutting-Sheath die starken Verwachsungen von der Elektrodenleitung ablöst;
f) Verschieben des Cutting-Sheath in proximaler Richtung in Bezug zum Locking-Sheath, so dass sich die Klemmvorrichtung in distale Richtung in die Aufnahme bewegt, wobei sich die Fixierung des Locking-Sheath von der implantierten Elektrodenleitung löst; und
g) Gegebenenfalls Wiederholen der Schritte c) bis f), bis die implantierte Elektrodenleitung frei von Verwachsungen ist und zusammen mit der Explantationsvorrichtung aus dem Körper entfernt werden kann.

Bei Elektrodenleitungen mit einem Führungsdrahtlumen besteht auch die Möglichkeit der Anwendung zusätzlich mit Hilfe eines Locking-Stylets. In diesem Fall umfasst das Explantationsverfahren mit Hilfe der Explantationsvorrichtung die folgenden Schritte:
a) Freipräparation der Elektrodenleitung und Abschneiden des Steckers der Elektrodenleitung;
b) Einführung und Verankerung des Locking Stylets im Führungsdrahtlumen der Elektrodenleitung;
c) Einführen des proximalen Endes der implantierten Elektrodenleitung mit darin eingeführtem Locking-Stylet in die distalen Lumenöffnungen des Cutting-Sheath und des Locking-Sheath der Explantationsvorrichtung, bei der sich die Klemmvorrichtung des Locking-Sheath im Ruhezustand in der Aufnahme des Cutting-Sheath befindet und damit Locking-Sheath (auch temporär) mit dem Cutting-Sheath geführt wird;
d) Vorschieben der Explantationsvorrichtung in Richtung des distalen Endes der implantierten Elektrodenleitung und dabei Ablösen von leicht anhaftenden Gewebeverwachsungen an der Elektrodenleitung;
e) bei Erreichen von starken Verwachsungen oder hohem Widerstand beim Vorschieben der Explantationsvorrichtung in Richtung des distalen Endes der Elektrodenleitung: Fixieren des Locking-Sheath zur Verhinderung einer Bewegung in Bezug zur implantierten Elektrodenleitung;
f) Verschieben des Cutting-Sheath in distaler Richtung in Bezug zum Locking-Sheath, so dass sich die Klemmeinrichtung in proximaler Richtung aus der Aufnahme bewegt, wobei sich das Locking-Sheath fest mit der implantierten Elektrodenleitung verklemmt und wobei die Ablöseeinrichtung des Cutting-Sheath die starken Verwachsungen von der Elektrodenleitung ablöst;
g) Verschieben des Cutting-Sheath in proximaler Richtung in Bezug zum Locking-Sheath, so dass sich die Klemmvorrichtung in distale Richtung in die Aufnahme bewegt, wobei sich die Fixierung des Locking-Sheath von der implantierten Elektrodenleitung löst; und
h) Gegebenenfalls Wiederholen der Schritte d) bis g), bis die implantierte Elektrodenleitung frei von Verwachsungen ist und zusammen mit der Explantationsvorrichtung aus dem Körper entfernt werden kann.

Während des Vorschiebens und Entfernen des Gewebes kann auch sowohl mit der kompletten Explantationsvorrichtung (bei leichten Verwachsungen) als auch nur mit dem Cutting-Sheath eine Drehbewegung um die Längsachse vorgenommen werden, um einen zusätzlichen Ablöseeffekt zu bewirken. Ebenso kann der Ablöseeffekt durch mehrmalig wiederholtes kurzes Vor- und Zurückschieben der Explantationsvorrichtung verstärkt werden.

## Patentansprüche

1. Explantationsvorrichtung zur Explantation implantierter Elektrodenleitungen (3) mit einem distalen, in Richtung des Explantationsortes weisenden Ende und einem proximalen, in Richtung des Operateurs weisenden Ende, wobei die Explantationsvorrichtung umfasst:
- ein Locking-Sheath (1) zur lösbaren Befestigung an einer implantierten Elektrodenleitung, umfassend einen schlauch- oder rohrförmigen Körper (16) mit einem proximalen und distalen Ende (16A, 16B) und einem Lumen (17) entlang einer Längsachse, welches sowohl am distalen als auch am proximalen Ende eine Öffnung aufweist, und weiter umfassend mindestens eine Klemmvorrichtung (11) am oder in der Nähe des distalen Endes, wobei die Klemmvorrichtung aus einem Ruhezustand in einen gespannten Zustand und zurück in den Ruhezustand überführbar ist; und
- ein Cutting-Sheath (2) zur Entfernung von verwachsenem Gewebe, umfassend einen schlauch- oder rohrförmigen Körper (23) mit einem proximalen und einem distalen Ende (23A, 23B) mit einem Lumen (25) entlang einer Längsachse, wobei das Lumen sowohl am proximalen als auch am distalen Ende eine Öffnung aufweist, und weiter umfassend eine Ablöseeinrichtung (21) am oder in der Nähe des distalen Endes, wobei das Cutting-Sheath entlang der Längsachse frei in Bezug zum Locking-Sheath (1) beweglich ist;
wobei das Cutting-Sheath (2) mindestens eine Aufnahme (22) für die Klemmvorrichtung des Locking-Sheath umfasst, welche so gestaltet ist, dass die Klemmvorrichtung in ihrem Ruhezustand darin aufgenommen ist.

2. Explantationsvorrichtung nach Anspruch 1, **dadurch gekennzeichnet, dass** zur lösbaren Befestigung an einer implantierbaren Elektrodenleitung die mindestens eine Klemmvorrichtung (11) des Locking Sheath (1) im gespannten Zustand eine Befestigung bewirkt und im Ruhezustand die Befestigung gelöst ist, wobei sich die Klemmvorrichtung im gespannten Zustand nicht in der Aufnahme (22) und in Längsrichtung verschoben zu dieser im Lumen (25) des Cutting-Sheath (2) befindet.

3. Explantationsvorrichtung nach Anspruch 1 oder 2, **dadurch gekennzeichnet, dass** die Klemmvorrichtung aus einem Ruhezustand in einen gespannten Zustand und/oder zurück in den Ruhezustand durch Federkraft überführbar ist.

4. Explantationsvorrichtung nach einem der Ansprüche 1 bis 3, **dadurch gekennzeichnet, dass** das Locking-Sheath (1) mit dem Cutting-Sheath (2) führbar ist, wenn sich die mindestens eine Klemmvorrichtung (11) in der mindestens einen Aufnahme (22) befindet.

5. Explantationsvorrichtung nach einem der Ansprüche 1 bis 3, **dadurch gekennzeichnet, dass** die mindestens eine Klemmvorrichtung (11) und die mindestens eine Aufnahme (22) je mindestens eine radiale Erstreckungskomponente umfasst.

6. Explantationsvorrichtung nach einem der Ansprüche 1 bis 4, **dadurch gekennzeichnet, dass** die mindestens eine Klemmvorrichtung (11) eine Spannzange ist, welche mindestens eine, vorzugsweise vier, in Richtung der Längsachse erstreckende Spannlaschen (12) umfasst, wobei jede Spannlasche einen ersten Abschnitt (13), der an einem ersten Ende am Locking-Sheath angebracht ist, und einen zweiten Abschnitt (14) mit einem radial in Richtung Längsachse ragendes Ende (15) aufweist.

7. Explantationsvorrichtung nach Anspruch 6, **dadurch gekennzeichnet, dass** der erste Abschnitt (13) der Spannlasche (12) im Ruhezustand eine von seinem ersten Ende zu einem zweiten Ende hin radial von der Längsachse weg gerichtete Erstreckungskomponente aufweist, und der zweite Abschnitt (14) am zweiten Ende des ersten Abschnittes angebracht ist und zum nach innen ragenden Ende (15) hin eine radial zur Längsachse gerichtete Erstreckungskomponente aufweist.

8. Explantationsvorrichtung nach Anspruch 6 oder 7, **dadurch gekennzeichnet, dass** das nach innen ragende Ende (15) im gespannten Zustand in das Lumen (17) ragt.

9. Explantationsvorrichtung nach einem der Ansprüche 6 bis 8, **dadurch gekennzeichnet, dass** das nach innen ragende Ende (15) zum Ende hin abgerundet, eckig angeschliffen, als Schneide angeschliffen, aufgeraut und/oder mit einer rutschfesten Beschichtung versehen ist.

10. Explantationsvorrichtung nach einem der Ansprüche 1 bis 9, **dadurch gekennzeichnet, dass** die mindestens eine Aufnahme (22) einen proximalen Wandungsabschnitt (24) und einen distalen Wandungsabschnitt (27) mit je einer Erstreckungskomponente in radialer Richtung umfasst, wobei in Richtung distales Ende des Cutting-Sheath (2) hin der proximale Wandungsabschnitt eine radial von der Längsachse weg gerichtete Richtungskomponente aufweist und der distale Wandungsabschnitt eine radial zur Längsachse hin gerichtete Richtungskomponente aufweist.

11. Explantationsvorrichtung nach Anspruch 10, **dadurch gekennzeichnet, dass** mindestens einer der beiden Wandungsabschnitte (24, 27) je eine Erstreckungsrichtung parallel zu den ersten bzw. zu den zweiten Abschnitten (13, 14) der mindestens einen Spannlasche (12) der mindestens einen Klemmvorrichtung (11) aufweist.

12. Explantationsvorrichtung nach einem der Ansprüche 1 bis 10, **dadurch gekennzeichnet, dass** das Locking-Sheath (1) radial innerhalb des Cutting-Sheath (2) in dessen Lumen (25) beweglich geführt ist und dass eine implantierte Elektrodenleitung (3) radial innerhalb des Locking-Sheath in dessen Lumen (17) einführbar ist, wobei die Klemmvorrichtung (11) durch Verschieben aus der Aufnahme (22) in das im Innendurchmesser an den Körper (16) des Locking-Sheath angepasste Lumen (25) in den gespannten Zustand überführbar ist und die Befestigung einer Elektrodenleitung bewirkt.

## Claims

1. Explantation device for explantation of implanted electrode leads (3), with a distal end pointing in the direction of the explantation site and a proximal end pointing in the direction of the operating surgeon, the explantation device comprising:
- a locking sheath (1) for detachable fastening to an implanted electrode lead, comprising a hose-shaped or tubular body (16) with a proximal and a distal end (16A, 16B) and a lumen (17) along a longitudinal axis, the lumen having an opening at both its distal end and at its proximal end, the locking sheath (1) further comprising, at or near the distal end, at least one gripping device (11) that can be changed from a free state into a tensioned state and back into the free state; and
- a cutting sheath (2) for removal of scar tissue, comprising a hose-shaped or tubular body (23) with a proximal and a distal end (23A, 23B) with a lumen (25) along a longitudinal axis, the lumen having an opening at both its proximal end and at its distal end, the cutting sheath (2) further comprising, at or near the distal end, a separation device (21), the cutting sheath being freely movable with respect to the locking sheath along the longitudinal axis (1);
wherein
the cutting sheath (2) comprises at least one receptacle (22) for the gripping device of the locking sheath, this receptacle (22) being shaped so that when the gripping device is in its free state the gripping device is held in the receptacle (22).

2. An explantation device according to claim 1, **characterized in that** for detachable fastening to an implantable electrode lead, the at least one gripping device (11) of the locking sheath (1) causes, when it is in the tensioned state, a fastening, and, when it is in the free state, a release of the fastening, of the gripping device, when in the tensioned state, not being located in the receptacle (22) and being displaced from it in the longitudinal direction within the lumen (25) of the cutting sheath (2).

3. An explantation device according to claim 1 or 2, **characterized in that** the gripping device can be changed by spring force out of a free state into a tensioned state and/or back into the free state.

4. An explantation device according to any one of claims 1 through 3, **characterized in that** the locking sheath (1) can be guided with the cutting sheath (2) if the at least one gripping device (11) is located in the at least one receptacle (22).

5. An explantation device according to any one of claims 1 through 3, **characterized in that** the at least one gripping device (11) and the at least one receptacle (22) each comprises at least one component extending in the radial direction.

6. An explantation device according to any one of claims 1 through 4, **characterized in that** the at least one gripping device (11) is a collet chuck that comprises at least one, preferably four, jaws (12) extending in the direction of the longitudinal axis, each jaw having a first section (13), that is at a first end of locking sheath, and a second section (14) with an end projecting radially in the direction of the longitudinal axis (15).

7. An explantation device according to claim 6, **characterized in that** in the free state the first section (13) of the jaw (12) has, in the direction from its first end to a second end, a component extending radially away from the longitudinal axis, and the second section (14) is at the second end of the first section and has, in the direction toward the inward projecting end (15), a component extending radially toward the longitudinal axis.

8. An explantation device according to claim 6 or 7, **characterized in that** in the tensioned state the inward projecting end (15) projects into the lumen (17).

9. An explantation device according to any one of claims 6 through 8, **characterized in that** the inward-projecting end (15) is, toward the end, rounded, ground at an angle, sharpened in the form of a cutting edge, roughened, and/or provided with a nonslip coating.

10. An explantation device according to any one of claims 1 through 9, **characterized in that** the at least one receptacle (22) comprises a proximal wall section (24) and a distal wall section (27), each with a component extending in the radial direction, the proximal wall section having, in the direction of the distal end of the cutting sheath (2), a direction component directed radially away from the longitudinal axis, and the distal wall section having a direction component directed radially toward the longitudinal axis.

11. An explantation device according to claim 10, **characterized in that** at least one of the two wall sections (24, 27) each has an extension direction parallel to the first or to the second section(s) (13, 14) of the at least one jaw (12) of the at least one gripping device (11).

12. An explantation device according to any one of claims 1 through 10, **characterized in that** the locking sheath (1) is movably guided radially within the lumen (25) of the cutting sheath (2) and that an implanted electrode lead (3) can be radially inserted into the lumen (17) of the locking sheath, it being possible to change the gripping device (11) into the tensioned state by moving it out of the receptacle (22) into the lumen (25) whose inside diameter is adapted to the body (16) of the locking sheath, causing it to fasten to an electrode lead.

## Revendications

1. Dispositif d'explantation destiné à l'explantation de lignes d'électrodes (3) implantées, avec une extrémité distale, pointée en direction d'un lieu d'explantation et une extrémité proximale pointée en direction d'un opérateur, où le dispositif d'explantation comprend :
- une gaine de verrouillage (1) pour la fixation de manière amovible sur une ligne d'électrode implantée, comprenant un corps (16) en forme de tuyau ou de tube avec une extrémité proximale et une extrémité distale (16A, 16B), et une lumière (17) le long d'un axe longitudinal, laquelle présente une ouverture à la fois à l'extrémité distale et à l'extrémité proximale, et comprenant en outre au moins un dispositif de serrage (11) sur ou au voisinage de l'extrémité distale, où le dispositif de serrage peut être transféré d'un état de repos vers un état contraint et de retour vers l'état de repos ; et
- une gaine de découpe (2) pour le retrait de tissus de cicatrisation, comprenant un corps (23) en forme de tuyau ou de tube avec une extrémité proximale et une extrémité distale (23A, 23B), avec une lumière (25) le long d'un axe longitudinal, où la lumière présente une ouverture à la fois à l'extrémité distale et à l'extrémité proximale, et comprenant en outre un dispositif de détachement (21) sur ou à proximité de l'extrémité distale, où la gaine de découpe est mobile le long de l'axe longitudinal par rapport à la gaine de verrouillage (1) ;
où la gaine de découpe (2) comprend au moins un logement (22) pour le dispositif de serrage de la gaine de verrouillage, lequel est conçu de telle manière que le dispositif de serrage y est logé dans son état de repos.

2. Dispositif d'explantation selon la revendication 1, **caractérisé en ce que**, pour la fixation amovible sur une ligne d'électrode implantable, l'au moins un dispositif de serrage (11) de la gaine de verrouillage (1) provoque une fixation à l'état contraint et la fixation est libérée à l'état de repos, où le dispositif de serrage, à l'état contraint, ne se trouve pas dans le logement (22) et est déplacé dans la direction longitudinale par rapport à celui-ci dans la lumière (25) de la gaine de découpe (2).

3. Dispositif d'explantation selon la revendication 1 ou 2, **caractérisé en ce que** le dispositif de serrage peut être transféré d'un état de repos vers un état contraint, et/ou de retour vers l'état de repos par une force de ressort.

4. Dispositif d'explantation selon l'une des revendications 1 à 3, **caractérisé en ce que** la gaine de verrouillage (1) peut être guidée avec la gaine de découpe (2) lorsque l'au moins un dispositif de serrage (11) se trouve dans l'au moins un logement (22).

5. Dispositif d'explantation selon l'une des revendications 1 à 3, **caractérisé en ce que** l'au moins un dispositif de serrage (11) et l'au moins un logement (22) comprennent chacun au moins une composante d'extension radiale.

6. Dispositif d'explantation selon l'une des revendications 1 à 4, **caractérisé en ce que** l'au moins un dispositif de serrage (11) est une pince de serrage, laquelle comprend au moins une, de préférence quatre, pattes de serrage (12) s'étendant en direction de l'axe longitudinal, où chaque patte de serrage présente une première partie (13) qui est rapportée à une première extrémité sur la gaine de verrouillage, et une deuxième partie (14) avec une extrémité (15) s'élevant radialement en direction de l'axe longitudinal.

7. Dispositif d'explantation selon la revendication 6, **caractérisé en ce que** la première partie (13) de la patte de serrage (12), à l'état de repos, présente une composante d'extension orientée radialement à partir de sa première extrémité vers sa deuxième extrémité, en s'éloignant de l'axe longitudinal, et la deuxième partie (14) est rapportée à la deuxième extrémité de la première partie et présente, vers l'extrémité (15) s'élevant vers l'intérieur, une composante d'extension orientée radialement par rapport à l'axe longitudinal.

8. Dispositif d'explantation selon la revendication 6 ou 7, **caractérisé en ce que** l'extrémité (15) s'élevant vers l'intérieur pénètre dans la lumière (17) à l'état contraint.

9. Dispositif d'explantation selon l'une des revendications 6 à 8, **caractérisé en ce que** l'extrémité (15) s'élevant vers l'intérieur est arrondie vers l'extrémité, est taillée en carré, est taillée sous forme d'un tranchant, est dépolie et/ou est munie d'un revêtement antidérapant.

10. Dispositif d'explantation selon l'une des revendications 1 à 9, **caractérisé en ce que** l'au moins un logement (22) comprend une partie de paroi proximale (24) et une partie de paroi distale (27) avec chacune une composante d'extension en direction radiale, où, en direction de l'extrémité distale de la gaine de découpe (2), la partie de paroi proximale présente une composante de direction orientée radialement en s'éloignant de l'axe longitudinal, et la partie de paroi distale présente une composante de direction orientée radialement par rapport à l'axe longitudinal.

11. Dispositif d'explantation selon la revendication 10, **caractérisé en ce qu'**au moins l'une des deux parties de paroi (24, 27) présente chaque fois une direction d'extension parallèle par rapport à la première partie, respectivement par rapport à la deuxième partie (13, 14) de l'au moins une patte de serrage (12) de l'au moins un dispositif de serrage (11).

12. Dispositif d'explantation selon l'une des revendications 1 à 10, **caractérisé en ce que** la gaine de verrouillage (1) est guidée de manière mobile radialement à l'intérieur de la gaine de découpe (2) dans sa lumière (25) et qu'une ligne d'électrode (3) implantée peut être insérée à l'intérieur de la gaine de verrouillage dans sa lumière (17), où le dispositif de serrage (11) peut être transféré dans l'état contraint par un glissement à partir du logement (22) dans la lumière (25) adaptée au niveau du diamètre intérieur au corps (16) de la gaine de verrouillage et provoque la fixation d'une ligne d'électrode.
